# EUROPÄISCHE PATENTSCHRIFT

(11) **EP 1 181 383 B1**
(45) Veröffentlichungstag und Bekanntmachung des Hinweises auf die Patenterteilung: **20.10.2004**
(21) Anmeldenummer: 00931141.6
(22) Anmeldetag: 09.05.2000
(51) Int. Cl.: C12P 7/02, C12P 1/02, C12M 1/00, B01J 13/04

(54) **VERFAHREN UND VORRICHTUNG ZUR IN-SITU EXTRAKTION**
METHOD AND DEVICE FOR IN-SITU EXTRACTION
PROCEDE ET DISPOSITIF D'EXTRATION IN-SITU

(30) Priorität: 28.05.1999 DE 19924701
(43) Veröffentlichungstag der Anmeldung: 27.02.2002
(73) Patentinhaber: INOTECH AG, 5605 Dottikon (CH)
(72) Erfinder: STARK, Daniel, CH-8600 Dübendorf (CH); MARISON, Ian, CH-1026 Echandens (CH); VON STOCKAR, Urs, CH-1095 Lutry (CH); HEINZEN, Christoph, CH-8702 Zollikon (CH)
(74) Vertreter: Hiebsch, Gerhard F., Dipl.-Ing.
(86) Internationale Anmeldenummer: PCT/EP2000/004120
(87) Internationale Veröffentlichungsnummer: WO 2000/073485

(56) Entgegenhaltungen:
- WO-A-96/28247
- DE-A- 3 718 934
- DATABASE BIOSIS [Online] BIOSCIENCES INFORMATION SERVICE, PHILADELPHIA, PA, US; 1987 BARROS M R A ET AL: "PRODUCTION OF ETHANOL BY IMMOBILIZED SACCHAROMYCES-BAYANUS IN AN EXTRACTIVE FERMENTATION SYSTEM" Database accession no. PREV198784024496 XP002147700 & BIOTECHNOLOGY AND BIOENGINEERING, Bd. 29, Nr. 9, 1987, Seiten 1097-1104, ISSN: 0006-3592
- ANTONELLI ANDREA ET AL: "Yeast influence on volatile composition of wines." JOURNAL OF AGRICULTURAL AND FOOD CHEMISTRY, Bd. 47, Nr. 3, März 1999 (1999-03), Seiten 1139-1144, XP002147699 ISSN: 0021-8561
- STARK DANIEL ET AL: "Comparison of different in situ product-removal methods to enhance the production of the aroma compound 2-phenylethanol." ABSTRACTS OF PAPERS AMERICAN CHEMICAL SOCIETY, Bd. 219, Nr. 1-2, März 2000 (2000-03), Seite AGFD 13 XP000946141 219th Meeting of the American Chemical Society.;San Francisco, California, USA; March 26-30, 2000 ISSN: 0065-7727

## Beschreibung

Die vorliegende Erfindung betrifft ein Verfahren zur In-Situ-Extraktion nach dem Oberbegriff des Patentanspruches 1 sowie zur In-Situ-Zuführung nach dem Oberbegriff des Patentanspruches 12.

Derartige biotechnologische Verfahren und zugehörige Vorrichtungen sind aus dem Stand der Technik allgemein bekannt; sie dienen der Herstellung einer Vielzahl von Produkten mit Hilfe von jeweils geeigneten Mikroorganismen. Ein typisches Beispiel ist das Erzeugen von Fermentationsprodukten, ein anderes der Einsatz auch sonstiger biologischer Zellen zur Produktgewinnung.

Der wesentliche Nachteil derartiger biotechnologischer Prozesse besteht jedoch darin, dass das erzeugte Produkt üblicherweise in verdünnter Form vorliegt, nämlich etwa als Lösung innerhalb des wässrigen Fermentationsmediums. Hier entsteht dann das Problem der Produktinhibition, nämlich eine wachsende Konzentration des erzeugten Produktes in der Lösung, die dazu führt, dass die Produktionsrate durch den Mikroorganismus mit entsprechend zunehmender Produktkonzentration abnimmt. Dieser Inhibitionseffekt führt dazu, dass der Mikroorganismus bei Überschreiten einer kritischen Konzentration des erzeugten Produktes im Fermentationsmedium inaktiv wird.

Um die nachteilige Wirkung der Produktinhibition zu überwinden, gibt es im Stand der Technik zahlreiche Ansätze, das biotechnologisch erzeugte Produkt aus etwa dem Fermentationsmedium während des Fortschreitens des Fermentationsprozesses zu entfernen, so dass zu keinem Zeitpunkt eine nachteilige bzw. kritische Konzentration erreicht wird; man spricht von der In-Situ-Produktgewinnung (ISPR = In-Situ-Product-Recovery). Mit einem derartigen, geeigneten ISPR-Prozess kann die Funktionsweise des Mikroorganismus mit hoher Produktionsrate für einen relativ langen Zeitraum sichergestellt werden, und insbesondere bietet ein solcher Produkt-Gewinnungsprozess auch zahlreiche Ansätze, die Gewinnungskosten günstig zu gestalten.

Ein solcher, bekannter Prozess zur ISPR besteht darin, eine als Extraktionsmittel für das jeweilige gewünschte Produkt wirkende Flüssigkeit vorzusehen, die selbst unlöslich bzw. unvermischbar mit einem wässrigen Fermentationsmedium ist. Durch die Affinität des Extraktionsmittels findet eine Aufteilung des gewonnenen Produktes statt, so dass die Produktkonzentration in der wässrigen Phase vermindert wird. Eine derartige Flüssig-Flüssig-Extraktion gilt als besonders ökonomisch und kostengünstig, bringt jedoch selbst auch Probleme.

Zum einen ist die Auswahl eines geeigneten, Wasser- unvermischbaren Lösungs- und Extraktionsmittels dadurch beschränkt, dass viele derartige Mittel im Hinblick auf die Mikroorganismen toxisch wirken. Zum zweiten bildet sich oft eine Emulsion zwischen dem wässrigen Fermentationsmedium und der auch als organische Phase bezeichneten Extraktionsmittellösung, da dieses Lösungsmittel, im direkten Kontakt mit den Zellen, Makromoleküle aus den Zellwänden herauslöst, die dann wiederum als Emulsionsbildner wirken. Die Folgen sind Probleme bei einer nachfolgenden Trennung, einer Verstopfung der Prozessanlagen usw., und deren Vermeidung führt insbesondere bei großtechnischen Anlagen zu hoher Prozesskomplexität, die wiederum einer ökonomischen Verwertung entgegensteht.

Aus der DE 37 18 934 A1 ist ein Verfahren zum Herstellen von aus einer Alginatschale und einem Kern aus einem biologisch aktiven Material oder einer öligen Substanz bestehenden Alginat-Perlen mit einer Größe im Millimeter-Bereich bekannt. Bei diesem werden gleichzeitig getrennte Ströme des Kernmaterials durch eine Rohrleitung und eines Gases durch eine andere Rohrleitung zugeführt, die gebildeten Tropfen des Alginates in eine Koagulierlösung eingetropft und die entstandenen Perlen zur Härtung ihrer Schale in der Koagulierlösung belassen. Das Kernmaterial soll eine Suspension von Zellen, Organellen oder eines teilchenförmigen biologisch aktiven Materials in einer Lösung eines Präpolymeren sein und das Präpolymere -- beispielsweise Polyacrylamidhydrazid (PAAH) -- anschließend polymerisiert werden. Dazu wird darauf hingewiesen, dass Alginat-Schalen ebenso als Behältnis für Öle, wie Pflanzenöl, Paraffinöl oder Suspensionen oder Emulsionen auf basis eines hydrophoben, wasserunlöslichen Mediums verwendet werden können. Die dazu angebotene Vorrichtung soll drei einzeln einstellbare - bevorzugt koaxiale - Rohrleitungen sowie Einrichtungen zum Einstellen ihres Abstandes zueinander an einer gemeinsamen koaxialen Auslass-Öffnung aufweisen, wobei die innere und die zweite Rohrleitung als Zuleitungen für das den Kern bildende Material bzw. die Alginat-Lösung dienen und die dritte Rohrleitung eine Gasleitung ist.

Eine Vorrichtung zur sterilen Verkapaelung von mikrobiellen, pflanzlichen und tierischen Zellen und von biologischen und chemischen Substanzen in kleine, im wesentlichen kugelförmige Teilchen im Bereich der Mikrometerskala ist der WO 96/28247 zu entnehmen. Bei dieser werden die Teilchen in einem von der Umgebung steril getrennten Gefäß durch Vibration eines Immobilisierungsgemisches erzeugt darin verarbeitet und angereichert. Das Immobilisierungsgemisch besteht aus einer polymeren Matrix sowie dem zu verkapselnden Material und wird zum einen durch einen mechanischen Vorschub mit konstantem Volumenstrom durch ein Rohr und eine Düse gefördert, die zusammen einen sog. Hold-up von < 0,5 ml aufweisen und mit einer Frequenz von 100 bis 2000 Hz vibriert werden sowie zum anderen in Schwingungen von 100 bis 2000 Hz versetzt und dadurch nach der Düse in annähernd gleich große Teilchen getrennt.

Im Dokument "Production of Ethanol by immobilized Saccharomyces-bayanus in an extractive fermentation system "aus Biotechnology und bioengineering Vol. 29, 1987, Nr. 9, Seite 1097-1104 wird ein Extraktionsverfahren unter Verwendung von organischen Lösungmitteln als Extraktionsmittel beschrieben. Dabei werden die verwendeten Hefezellen immobilisiert und mit dem Extraktionsmittel in kontakt gebracht.

Ein weiteres, aus dem Stand der Technik bekanntes Verfahren besteht darin, das das gelöste Produkt enthaltende Fermentationsmedium von der Extraktionslösung durch eine Membran zu trennen. Insbesondere großtechnische Prozesse verlangen jedoch nach einer großen Membranfläche, und durch die auf einen speziellen Prozess (d.h. ein spezifisches, erzeugtes Produkt bzw. einen Mikroorganismus) im Hinblick auf ihre Transmissionseigenschaften spezifisch einzustellende Membran ist oftmals eine dezidierte Anlage für einen Prozess, verbunden mit hohem Investitionsaufwand, nötig, und insbesondere ein Prozesswechsel zu einem anderen biotechnologisch zu erzeugenden Produkt ist durch eine solche Anlage stark erschwert oder gar unmöglich. Darüber hinaus weisen bekannte Membranen zudem den Nachteil einer zunehmenden Verstopfung bzw. Blockierung der Membraneinheiten durch langkettige Moleküle auf, so dass auch hier großtechnisch lauffähige Prozessanlagen äußerst komplex und dementsprechend teuer sind.

Aufgabe der vorliegenden Erfindung ist es daher, ein Verfahren zur In-Situ-Extraktion (Gewinnung) von biotechnologisch erzeugten Produkten zu schaffen, welches ökonomisch und mit geringem prozess-spezifischen Aufwand zu realisieren ist, an Anlagen und Apparate geringe Anforderungen stellt und im Hinblick auf seine Extraktionseigenschaften des zu erzeugenden Produktes aus einem Mikroorganismen-Medium verbessert ist.

Die Aufgabe wird durch ein Verfahren zur In-Situ-Extraktion eines mittels eines Mikroorganismus oder einer biologischen Zelle erzeugten Produktes, insbesondere eines Fermentationsproduktes, mit den Schritten:
- Vorsehen einer biologischen Zell- bzw. Mikroorganismenkultur in flüssiger, bevorzugt wässriger Phase in einem Reaktionsbehälter;
- Hinzufügen eines Substrates, insbesondere einer Amminosäure, zu der flüssigen Phase, das so ausgewählt und/oder ausgebildet ist, dass durch Wirkung der Zellen oder Mikroorganismen das erzeugte Produkt in der flüssigen Phase entsteht, und
- Trennen des erzeugten Produktes von der flüssigen Phase in eine organische Phase mittels eines Lösungsmediums,
   gelöst, wobei der Schritt des Trennens die Schritte aufweist:

- Verkapseln eines organischen Lösungsmittels als Lösungsmedium außerhalb des Reaktionsbehälters in eine Mehrzahl von Kapseln mit einer Kapselwand, die eine Sperrwirkung für die Zellen bzw. Mikroorganismen sowie eine Durchlässigkeit für das erzeugte Produkt aufweist, und
- Einbringen der Mehrzahl von Kapseln in den Reaktionsbehälter und/oder in die flüssige Phase.

Weiterhin wird eine Vorrichtung zur In-Situ-Extraktion eines mittels eines Mikroorganismus oder einer biologischen Zelle erzeugten Produktes, insbesondere eines Fermentationsproduktes, zur Durchführung des vorabbeschriebenen Verfahrens, mit einem Reaktorbehälter zur Aufnahme einer biologischen Zell- bzw. Mikroorganismenkultur in flüssiger Phase sowie einer Substanz, die in Verbindung mit der Zell- bzw. Mikroorganismenkultur das erzeugte Produkt entstehen läßt, vorgeschlagen wobei außerhalb des Reaktorbehälters vorgesehene Mittel zum Verkapseln eines organischen Lösungsmittels zum Trennen des erzeugten Produktes von der flüssigen Phase in eine organische Phase vorgesehen sind, wobei die Mittel zum Verkapseln eine Düsenanordnung aus einer ersten, inneren Düse für das organische Lösungsmittel sowie einer zweiten, äußeren und konzentrisch angeordneten Düse für ein flüssiges Kapselwandmaterial aufweisen und diese Doppeldüse mit einem Vibrator mechanisch verbunden ist, um die austretenden Kapseln zu erzeugen, sowie Mittel zum Einbringen von durch die Verkapselungsmittel erzeugten Kapseln in den Reaktorbehälter vorgesehen sind. Vorteilhafte Weiterbildungen der Erfindung sind in den Unteransprüchen beschrieben.

Erfindungsgemäß vorteilhaft wird, in völliger Abkehr von bekannten In-Situ-Gewinnungsverfahren, der Ansatz gewählt, das Lösungsmittel in kapselierter Form in die sowohl die Mikroorganismen als auch das durch diese erzeugte Produkt aufweisende Lösung hineinzubringen, und durch erfindungsgemäß speziell ausgestaltete Kapselwände, nämlich mit selektiver Durchlasswirkung für das erzeugte Produkt, wird die gewünschte Trennwirkung auf einfache und effiziente Weise erreicht.

Darüber hinaus ist, durch entsprechende Wahl eines jeweiligen Kapselwandmaterials bzw. eines darin einzuschließenden Lösungsmittels, der Prozess flexibel und mit geringstem Aufwand auf andere Gewinnungsverfahren, zu erzeugende Produkte und Mikroorganismen ein- und umstellbar, ohne dass es etwa speziell angepasster Filtermembranen od.dgl. bedarf; insbesondere kleinere Chargen, die keine spezifisch eingerichtete Fertigungsanlage rechtfertigen, werden daher überhaupt erst ökonomisch realisierbar.

Erfindungsgemäß wird damit ein neuer Prozess für die In-Situ-Gewinnung von Produkten und Verbindungen geschaffen, die durch lebende Zellen produziert werden. Durch die bevorzugt als Gel bzw. Hydrogel-Kapsel realisierte Verkapselung wird die organische Phase mit dem Lösungsmittel nicht in direkten Kontakt mit den Mikroorganismen gebracht, so dass das erfindungsgemäße Verfahren sämtliche Vorteile bekannter, mittels Filtermembranen od.dgl. realisierter ISPR-Prozesse besitzt, gleichzeitig jedoch demgegenüber erhebliche Kosten- und Flexibilitätsvorteile realisiert.

In diesem Zusammenhang ist zu beachten, dass die vorliegende Erfindung auch für allgemeine Biokatalysatoren zur Produktgewinnung, etwa Enzyme, anwendbar ist und insoweit die vorliegende Erfindung, über biologische Zellen und Mikroorganismen hinaus, auch generell Biokatalysatoren als Erzeugungsmittel für das erzeugte Produkt umfaßt.

Darüber hinaus bieten die Kapseln die Möglichkeit einer Wiederaufbereitung (Re-Konditionierung) durch Re-Extraktion des erzeugten Produktes (im weiteren auch Zielprodukt genannt) und nachfolgendes Weiterverwenden der Kapsel.

Ferner ist durch die vorliegende Erfindung die Möglichkeit geschaffen, mittels der Kapseln das eigentliche Substrat (die den Mikroorganismen zum Erzeugen des Endproduktes zuzugebende Substanz) überhaupt erst in den Prozess einzuführen; so existieren nämlich Prozesse, bei denen ein unkontrolliertes Hinzufügen der Substanz zu der flüssigen Phase eine toxische Wirkung auf die Mikroorganismenkultur haben kann, so dass es sinnvoll ist, diese Substanz in kapselierter Form, unterstützt durch die Membranwirkung der Kapselwand, die in diesem Fall die Substanz in die umgebende Lösung austreten läßt, als Transportmedium zu benutzen. Insoweit fällt unter die vorliegende Erfindung auch ein Verfahren zur In-Situ-Zuführung einer Substratverbindung od.dgl. zu einem biotechnologischen Prozess.

Besonders bevorzugt ist es im Rahmen der vorliegenden Erfindung, die Verkapselung des organischen Lösungsmittels (bzw. des Substrats/der Substanz im Fall der In-Situ-Zuführung) durch eine Verkapselungsvorrichtung durchzuführen, die flüssiges Kapselwandmaterial, z.B. Hydrogel, durch gepulste Zuführung zum Umschließen des Kapselinhaltes verwendet, woraufhin dann, etwa durch Einbringen in ein geeignetes Verfestigungsmedium, die (nach wie vor flüssige) Kapselwand verfestigt oder teilverfestigt wird.

Eine mögliche Realisierungsform der Erfindung sieht vor, die biologischen Zellen bzw. Mikroorganismen oder das Enzym nicht in flüssiger Phase in einem Reaktorbehälter vorzusehen, sondern diese durch Einbau in die Kapselwand zu immobilisieren und damit eine Nähe zwischen der organischen Phase im Kapselinneren und dem Biokatalysator herzustellen.

Die bevorzugt sphärischen Hydrogel-Kapseln können weiterbildungsgemäß auch eine mehrschichtige Kapselwand aufweisen, wodurch deren Membraneingenschaften zusätzlich und spezifischer kontrollierbar werden. Vorzugsweise liegt der Außendurchmesser bei der Mehrzahl der Kapseln im Bereich zwischen 0,1 und 2,5 mm, sowie deren Kapselwandstärke im Bereich zwischen 30 und 300 Mikrometern bevorzugt zwischen 5,2 und 250 Mikrometern.

Eine besonders vorteilhafte Eigenschaft der erzeugten Kapseln ist es zudem, geeignet autoklavier- bzw. sterilisierbar zu sein, so dass deren Verwendung insbesondere auf sterile Prozesse besonders günstig ist.

Im Ergebnis werden durch den erfindungsgemäßen Ansatz die aus dem Stande der Technik bekannten Nachteile in überaus effizienter und eleganter Weise überwunden, und die vorteilhaften Eigenschaften einer Matrixstruktur einer verfestigten Kapselwand werden benutzt, um die effiziente In-Situ-Trennung (bzw. Zuführung) zu ermöglichen.

Dabei bietet der erfindungsgemäße Prozess auch das Potential, als kontinuierlicher Prozess oder quasi-kontinuierlicher Prozess (mit aufeinanderfolgenden Batches) realisiert zu werden, indem nämlich entsprechende Reaktionsbehälter, Kapselierungseinrichtungen sowie Kapsel-Re-Extraktionseinheiten vorgesehen sind.

Weitere Vorteile, Merkmale und Einzelheiten ergeben sich aus der nachfolgenden Beschreibung bevorzugter Ausführungsbeispiele der Erfindung sowie anhand der Zeichnungen; diese zeigen in
- Fig. 1:: einen schematischen Prinzipaufbau eines Kapselierungssystems zur Verwendung mit der vorliegenden Erfindung;
- Fig. 2:: einen Messwertverlauf einer Produktkon- zentration in wässriger Fermentationslösung während der erfindungsgemäßen Behandlung mit in Mikrokapseln eingeschlossenem Lösungsmittel und

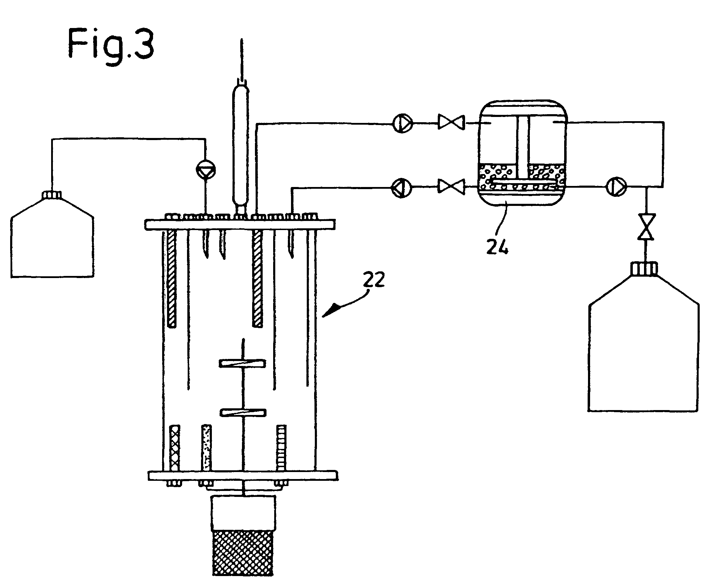

- Fig. 3:: eine schematische Prozessanlage zur In-Situ-Extraktion gemäß einer Ausführungsform der Erfindung und nachgeschalteter Re-Extraktion des Zielproduktes aus den Mikrokapseln.

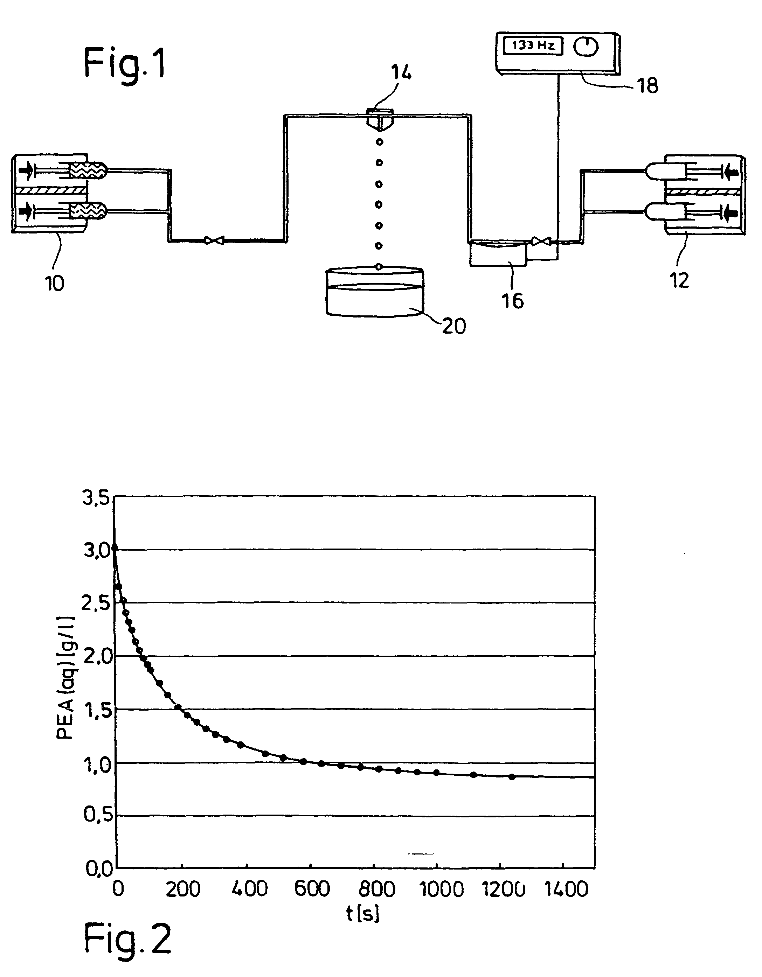

Gemäß einem ersten, bevorzugten Ausführungsbeispiel der Erfindung werden Mikrokapseln eingesetzt, die einen das organische Lösungsmittel aufweisenden Kapselkern (Nucleus) sowie eine Hydrogel-Kapselwand aufweisen. Dabei hat es sich herausgestellt, dass ein Düsensystem mit zwei Flüssigkeiten nahezu perfekt konzentrische, sphärische Kapseln mit gleichförmiger Größenverteilung, hier im Bereich zwischen 1,6 und 2,06 mm, wie in Fig. 1 gezeigt, gestattet.

Wie in Fig. 1 gezeigt, fließt die zu verkapselnde, organische Flüssigkeit (Nucleus) durch eine schematisch als Pumpe 10 gezeigten Fördereinheit zu einer inneren Düse, und flüssiges Kapselwandmaterial (Hülle) fließt mittels einer Pumpenanordnung 12 zu einer äußeren Düse einer gemeinsamen Düseneinheit 14.

Eine oszillierende Membraneinheit 16, die mittels eines Schwingungsgenerators 18 im vorliegenden Fall in periodische Schwingungen versetzt wird, ändert mit dieser Schwingungsfrequenz die Zuflussrate des flüssigen Kapselbandmaterials von der zweiten Pumpeneinheit 12 zur äußeren Düse der Düseneinheit 14, wodurch eine Kapselbildung -- noch mit weicher, unverfestigter Kapselwand -- erreicht wird. Diese erzeugten Kapseln fallen, wie in der Fig. 1 gezeigt, in einen Geliermittelbehälter 20 mit einer geeigneten Gelierlösung, welche eine Verfestigung der weichen Kapselwand, im vorliegenden Fall die Bildung eines Polyelektrolyt-Komplexes, bewirkt. Die Gelkapseln werden dadurch fest und damit im nachfolgenden Extraktionsprozess verwendbar. Die durch die Einrichtung gemäß Fig. 1 erzeugten Kapseln weisen typischerweise einen Kern (Nucleus) eines Durchmessers von 0.2 - 2 mm auf, wobei die Kapselwandstärke zwischen etwa 50 Mikrometern (für autoklavierte Kapseln) und etwa 230 Mikrometern (für nicht-autoklavierte Kapseln) liegen kann.

Grundsätzlich ist für die Kapselerzeugung durch Schwingungsanregung gemäß Fig. 1 jedes Poly-Anion oder Poly-Kation als Wandmaterial geeignet, wobei bevorzugte Polyelektrolyte Alginate, Chitosan oder Derivate von diesen sein können. Insbesondere weisen diese Stoffe den Vorteil auf, dass sie i.w. für die industriell einzusetzenden, nützlichen Mikroorganismen nicht toxisch und darüber hinaus preisgünstig sind.

Der Kapselkern (Nucleus) kann aus jedem beliebigen, geeigneten organischen Lösungsmittel oder einer Mischung von verschiedenen Lösungsmitteln bestehen, die bevorzugt unlöslich in Wasser sein sollten. Weiter bevorzugt kann das Lösungsmittel im Kapselkern einen Komplexbildner als Extraktionsmittel aufweisen, wobei insbesondere ternäre und quaternäre Amine als Komplexbildner für Carboxylsäuren günstig sind.

Die im Behälter 20 enthaltene Geliermittellösung besteht aus beliebigen, einfachen anorganischen Ionen oder Polyelektrolyten, die mit dem Polyelektrolyt der Kapselwand einen Komplex bilden können.

Besonders geeignet im Rahmen der vorliegenden Erfindung sind die beschriebenen Kapseln insbesondere auch zum Zuführen (Einbringen) eines Substratmaterials in eine die Kapseln umgebende Mikroorganismenlösung, wobei auch hier die Kapselwand als Membran für das in geeigneter organischer Phase gelöste Substrat wirkt.

Durch Einbringen der wie im Zusammenhang mit Fig. 1 beschriebenen, das Exktraktions-Lösungsmittel enthaltenden Kapseln in eine wässrige Fermentationslösung können die wesentlichen Nachteile bekannter In-Situ-Extraktionsverfahren vermieden werden, nämlich das unerwünschte Bilden einer Emulsion von Lösungsmittel und wässriger Lösung (hier verhindert durch die selektiv durchlässige Kapselwand), sowie die Notwendigkeit spezifisch ausgebildeter, unflexibler und aufwendiger Membranfilter. Die Verkapselung der organischen Phase in einem Hydrogel ermöglicht dagegen beträchtliche Flexibilität und macht, durch die mittels der Kapselwände einer Mehrzahl von Kapseln erreichbare, große Oberfläche und die gute Diffusionseignung einer Kapselwand das Verfahren insbesondere auch für kommerzielle Prozesse interessant.

Wie bei der bekannten, einfachen Flüssig-Flüssig-Extraktion aus einer Fermentationslösung ist die Wahl des (hier in der Hydrogel-Kapsel zu verkapselnden) Lösungsmittels kritisch für den Erfolg und die Leistungsfähigkeit des Prozesses. Das Lösungsmittel muss für das erzeugte Produkt (Zielprodukt) selektiv sein und sollte darüber hinaus eine hohe Aufnahmekapazität hierfür besitzen. Dabei bestimmt dann die gewünschte Aufteilung des Zielproduktes zwischen organischer (Lösungsmittel-) und wässriger Phase die Randbedingungen für Anzahl und Ausgestaltung der Mikrokapseln; so kann etwa das Zielprodukt so gut in der organischen Phase lösbar sein, dass dieses während des gesamten Fermentationsprozesses im Lösungsmittel gehalten wird, so daß die Zielprodukt-Konzentration zu keinem Zeitpunkt in der wässrigen Phase einen für die Mikroorganismen toxischen Pegel erreichen kann. Insbesondere in diesem Fall muss keine Re-Konditionierung der Mikrokapseln erfolgen, und diese können direkt in die Fermentationseinheit eingebracht werden. Wenn dagegen die Aufnahmekapazität des Lösungsmittels für das Zielprodukt nicht groß genug ist, kann möglicherweise eine Notwendigkeit bestehen, die Kapseln während des Fermentationsprozesses zu re-konditionieren, also das Produkt extern aus den Kapseln heraus zu extrahieren, entweder durch das bereits innerhalb der Kapsel benutzte Lösungsmittel, durch ein anderes Lösungsmittel oder gar eine wässrige Phase. Nach einer solchen Re-Extration wäre die Mikrokapsel wiederum in der Lage, das zu gewinnende inhibitorische Produkt aus der Fermentationslösung zu ziehen und kann zu diesem Zweck (zurück) in den Reaktionsbehälter gebracht werden. Insbesondere im Fall der Notwendigkeit einer solchen Re-Extraktion (mit mehreren Extraktions- und Re-Extraktionszyklen) können die Mikrokapseln außerhalb der Fermentationseinheit in einer geeigneten Extraktionseinheit behandelt werden, wobei, in einem ersten Schritt, die Kapseln mit dem inhibitorischen Zielprodukt beladen werden, und daraufhin dann außerhalb des Fermentationsbehälters durch Re-Extraktion das Zielprodukt herausgelöst wird. Um aus einem solchen Verfahren einen kontinuierlichen Prozess zu machen, bietet es sich an, die Extraktions- und Re-Extraktionsschritte in Form aufeinanderfolgender Batches einzurichten.

Vorteilhaft wird zudem die Lösung in der externen Re-Extraktionseinheit bewegt, so dass hier möglicherweise vorhandene Mikroorganismen keine Probleme, etwa durch Verstopfen des Systems, erzeugen, und ein getrenntes Rezyklieren der Zellen od.dgl. ist nicht notwendig.

Der zweite Anwendungsfall, wie vorstehend beschrieben, der erfindungsgemäßen Kapseln liegt in dem gesteuerten Zuführen von Stoffen in einen Biotransformationsprozess, indem die Mikrokapseln als Aufnahmebehälter mit definierter Auslasswirkung angesehen werden. Ein solcher Ansatz eignet sich insbesondere für Substratverbindungen oder Substratmaterial eines biotechnologischen Prozesses, das, im Hinblick auf die Mikroorganismen, selbst inhibitorisch wirken könnte, oder aber hinsichtlich der wässrigen Mikroorganismen-Lösung nur eine sehr niedrige Löslichkeit aufweist. In beiden Fällen würde der Kapseleinsatz für das Substrat zum Trennen zwischen der wässrigen Phase außerhalb der Kapseln und der organischen Phase innerhalb der Kapseln zu einer letztendlich gewünschten, notwendigen Konzentration des Substrats in der wässrigen Phase führen, so dass im Fall etwa einer Substrat-Inhibition die Mikroorganismen stets nur mit einer Konzentration konfrontiert werden, die unterhalb des toxischen Pegels liegt. Im zweiten Fall der niedrigen Substrat-Löslichkeit ermöglichen die Kapseln eine (ansonsten unmögliche) hohe Substratkonzentration in der umgebenden wässrigen Fermentationslösung.

Weitere Merkmale und Details der Erfindung sowie ein Nachweis für deren Leistungsfähigkeit und Brauchbarkeit ergeben sich aus der nachfolgenden Beschreibung von konkreten Beispielen und Rezepturen.

### Beispiel 1:

Dieses Beispiel zeigt die erfolgreiche Extraktion von 2-Phenylethanol (Fluka, CH-Buchs) aus einer wässrigen Lösung in Mikrokapseln, die mit Dibutylsebacat (Fluka, CH-Buchs) gefüllt sind. 3,6 ml/s einer 15-g/l Natrium-Alginatlösung (Fluka, CH-Buchs) sowie 1,7 ml/s von Dibutylsebacat werden durch eine Zweifachdüse (1 mm Auslassöffnung) in einem wie in Fig. 1 gezeigten Kapselierungssystem gefördert. Die Zuflussrate der Alginatlösung wurde mit einer Membran bei 133 Hz gepulst. Die gebildeten Kapseln fallen in eine Lösung aus 16 g/l CaCl₂•2H₂O (Fluka, CH-Buchs), wo sie dann gelieren. Nach dem Autoklavieren (20 Minuten bei 120°) weisen die Kapseln einen Außendurchmesser von 1.730 ± 30 µm sowie einen Innendurchmesser (organische Phase) von 1.610 ± 40 µm auf.

Um die Extraktionskinetik zu bestimmen, wurde eine 150 ml-Lösung von 3 g/l 2-Phenylethanol bei 400 U/min bewegt. 1.960 Dibutylsebacat-Mikrokapseln wurden hinzugefügt, und die Abnahme des 2-Phenylethanol in der wässrigen Phase wurde beobachtet; die Fig. 2 zeigt die Abnahme der 2-Phenylethanol-Konzentration in der wässrigen Lösung. Die Gesamt-Kapseloberfläche mit einer Oberflächengröße von 121 cm² weist einen Massentransferkoeffizienten k_{L} von 4,1•10⁻⁶ m/s auf; hierdurch erhält k_{L} ein Wert von 10,5 h⁻¹ im benutzten System.

### Beispiel 2:

Dieses Beispiel zeigt die erfolgreiche Extraktion von 2-Phenylethanol aus einer Fermentationslösung unter Benutzung eines Lösungsmittels in Mikrokapseln, die direkt im Reaktor vorgesehen sind. Die Biotransformation von L-Phenylalanin (L-Phe) in 2-Phenylethanol (PEA) wird durch den Stamm Saccharomyces bayanus CBS 426 realisiert.

Saccharomyces bayanus CBS 426 wurde mit dem folgenden, definierten Medium kultiviert: 6 g/l (NH₄)₂SO₄, 1,92 g/l (NH₁)₂HPO₄, 0,87 g/l KCl, 0,45 g/l MgSO₄•7H₂O, 0,28 g/l CaCl₂•2H₂O, 2,3 mg/l CuSO₄•SH₂O, 14,6 mg/FeCl₃•6H₂O, 9 mg/l ZnSO₄•7H₂O, 10,5 mg/l MnSO₄•H₂O, 30 mg/l Ca-Pantothenat, 60 mg/l Myo-Inositol, 6 mg/l Thiaminchlorid, 1,65 mg/l Pyridoxalhydrochlorid, 0,03 mg/l Biotin und 0,05 g/l Polypropylenglykol 2000. All diese Chemikalien sind durch die Fluka AG (CH-Buchs) erhältlich. Die Glukose- und L-Phenylalanin-Konzentrationen sind in ihrer jeweiligen Anwendung unten angegeben.

Die Konzentrationen von 2-Phenylethanol und L-Phenylalanin wurden mittels HPLC (Serie 1100, HP, USA) ermittelt. Zweifach destilliertes Wasser und Acetonitril wurden als Eluationsmittel eingesetzt, indem sie einen linearen Gradient bilden, der mit 100% Wasser beginnt und nach 12 Minuten 100% Acetonitril enthält. Eine Flussrate von 1 ml/min wurde durch die Kolonne (RP-18 endcapped 125 mm, Supersphere, Merck, Darmstadt, DE) geleitet und anschließend anhand eines UV-Detektors bei 254 nm analysiert. Glukose- und Ethanolkonzentrationen wurden ebenfalls mittels HPLC bestimmt, indem eine 0.005 M schwefelsäurehaltige wässrige Lösung isokratisch als Eluationsmittel diente (0.8 ml/min). Verwendet wurde dabei eine Ionenaustauscherkolonne (Supelcogel H, Supelco, USA) und ein RI-Detektor bei einer Temperatur von 50°C. Die Kohlendioxidkonzentration wurde mit einem akkustischen Gasanalysator (Brüel & Kjaer Typ 1311, Naerum, DK) bestimmt. Die Trockenmasse der Hefen wurde ermittelt, indem die Zellen durch Zentrifugation (9 Minuten bei 3500 U/min) abgetrennt wurden. Sie wurden durch Resuspension in Wasser gewaschen und nochmals abzentrifugiert. Anschliessend wurden die Zellen bei 100°C während 36 Stunden im Ofen getrocknet, bevor sie gewogen wurden.

Als Bioreaktor wurde ein 1.6 Liter Fermenter (Bioengineering KLF 2000, Wald, CH) eingesetzt, in welchem durch Zugabe einer 2 M NaOH-Lösung der pH auf einem konstanten Wert von 4 geregelt wird. Die Rührerdrehzahl wurde auf 250 U/min eingestellt und 1.3 Liter Luft wurden pro Minute durch den Reaktor geblasen. Die Chargenfermentation wurde mit 70 g/l Glukose und 6 g/l L-Phe in einem Arbeitsvolumen von 1200 ml gestartet. Zusätzlich wurden zu Beginn der Fermentation 185 g sterile Mikrokapseln mit einem äußeren Durchmesser von 1.76 mm dem Reaktionsmedium hinzugefügt. Die Kapseln enthielten zusammen ein totales Volumen von 145 ml Oleinsäure, welche als Extraktionsmittel eingesetzt wurde. Die Mikrokapseln wurden wie in Beispiel 1 beschrieben hergestellt. Nach 18 Stunden der Fermentation wurde zusätzlich zur wässrigen PEA-Konzentration (PEAaq) auch die PEA-Konzentration in der organischen innerhalb der Kapseln bestimmt (PEAorg), indem diese mit Hilfe einer Trinatriumcitrat-Lösung (Merck, USA) aufgelöst wurden. Aus diesen beiden PEA-Konzentrationen wurde die totale PEA-Konzentration berechnet (PEAtot).

L-Phe wurde durch S. bayanus zu PEA umgesetzt, welches gleichzeitig selektiv durch die Oleinsäure in die Mikrokapseln extrahiert wurde. PEA hat einen Verteilungskoeffizienten von 7 zwischen der organischen Phase und dem wässrigen Fermentationsmedium, und wird deshalb in den Kapseln akkumuliert. Das CO2-Signal, das bei der vollständigen Konsumation der vorhandenen Glukose abfiel, diente als Indikator für die erneute Glukosezugabe in den Zugaben 1,2 und 3 (jeweils 250 ml an 180g/l Glukose-Lösung), wodurch die PEA-Produktion aufrechterhalten wurde. Nach 28 Stunden Fermentationszeit wurde eine PEA-Konzentration in der organischen Phase von 17.5 g/l erreicht. Gleichzeitig konnte die wässrige PEA-Konzentration auf einem nicht inhibitorischen Niveau von weniger als 3g/l gehalten werden. Durch die Verkapselung des Lösungsmittels in den Kapseln wurde während der ganzen Fermentation, dessen direkten Kontakt mit den Hefezellen vermieden und dadurch keine Emulsion gebildet. Zusätzlich konnten am Ende der Fermentation die Kapseln auf einfache Weise abgeschöpft werden.

### Beispiel 3:

Dieses Beispiel zeigt die erfolgreiche In-Situ-Produktgewinnung in einem Fermentationsprozess mit Hilfe eines externen Extraktors, der mit lösungsmittelhaltigen Mikrokapseln gefüllt ist.

Wie im Beispiel 2, wird L-Phenylalanin zu 2-Phenylethanol durch S. bayanus transformiert. Das Kulturmedium sowie die Analysevorrichtungen sind im Beispiel 2 beschrieben. Die Anlage besteht aus einem 3,6 Liter Bioreaktor 22 (z.B. Bioengineering KLF 3000, CH-Wald), der mit einem Rührtank 24 eines Aufnahmevolumens von 1 Liter verbunden ist. Dieser Extraktor-Tank enthält 24.200 Mikrokapseln, die insgesamt 54 ml Dibutylsebactat enthalten. Während eines Extraktionszyklus wird die Fermentationslösung 10 Minuten durch den Reaktor 22 geleitet, und die Aufenthaltszeit der Zellen in der den Extraktionstank 24 aufweisenden externen Schleife beträgt 1 Minute. In einem zweiten Schritt werden 150 ml Dibutylsebctat 10 Minuten durch den Extraktor-Tank 24 zirkuliert, um das 2-Phenylethanol aus den Kapseln zu extrahieren. Nach dieser Re-Konditionierung der Kapseln beginnt der nächste Zyklus mit einer weiteren Batch-Extraktion des Zielproduktes aus der Fermentationslösung.

## Patentansprüche

1. Verfahren zur In-Situ-Extraktion eines mittels eines Mikroorganismus oder einer biologischen Zelle erzeugten Produktes, insbesondere eines Fermentationsproduktes, mit den Schritten:
- Vorsehen einer biologischen Zell- bzw. Mikroorganismenkultur in flüssiger, bevorzugt wässriger Phase in einem Reaktionsbehälter;
- Hinzufügen eines Substrates, insbesondere einer Amminosäure, zu der flüssigen Phase, das so ausgewählt und/oder ausgebildet ist, dass durch Wirkung der Zellen oder Mikroorganismen das erzeugte Produkt in der flüssigen Phase entsteht, und
- Trennen des erzeugten Produktes von der flüssigen Phase in eine organische Phase mittels eines Lösungsmediums,
**dadurch gekennzeichnet, dass**
der Schritt des Trennens die Schritte aufweist:
- Verkapseln eines organischen Lösungsmittels als Lösungsmedium außerhalb des Reaktionsbehälters in eine Mehrzahl von Kapseln mit einer Kapselwand, die eine Sperrwirkung für die Zellen bzw. Mikroorganismen sowie eine Durchlässigkeit für das erzeugte Produkt aufweist, und
- Einbringen der Mehrzahl von Kapseln in den Reaktionsbehälter und/oder in die flüssige Phase.

2. Verfahren nach Anspruch 1, **gekennzeichnet durch** die Schritte:
- Entfernen der Mehrzahl von Kapseln aus dem Reaktionsbehälter nach Ablauf einer vorbestimmten Zeit nach dem Einbringen und
- Freisetzen des erzeugten Produktes und/oder der organischen Phase **durch** Zerstören der Kapselwand oder Anwendung eines weiteren Lösungsmittels.

3. Verfahren nach Anspruch 1 oder 2, **dadurch gekennzeichnet, dass** der Schritt des Verkapselns die Schritte aufweist:
- Ausbringen des organischen Lösungsmittels durch eine erste Düse,
- gleichzeitiges Ausbringen eines flüssigen Kapselwandmaterials durch eine zweite konzentrisch angeordnete Düse,
- Zerteilung dieses Lösungsmittel-Kapselwandmaterial-Gemisches durch Überlagerung einer externen Schwingung in gleichgrosse Fraktionen, so dass eine Kapselbildung von durch das flüssige Kapselwandmaterial umschlossenem organischen Lösungsmittel entsteht, und
- Verfestigen des flüssigen Kapselwandmaterials nach der Kapselbildung.

4. Verfahren nach Anspruch 3, **dadurch gekennzeichnet, dass** das periodisch oszillierende Verändern in einem Frequenzbereich zwischen 30 und 4000 Hz.

5. Verfahren nach Anspruch 3 oder 4, **dadurch gekennzeichnet, dass** der Schritt des Verfestigens das Einbringen der Kapseln in eine eine Gelierungsreaktion bewirkende Gelierungslösung aufweist.

6. Verfahren nach einem der Ansprüche 1 bis 5, **gekennzeichnet durch** das Autoklavieren der Mehrzahl von Kapseln vor dem Einbringen in den Reaktionsbehälter.

7. Verfahren nach einem der Ansprüche 1 bis 6, **dadurch gekennzeichnet, dass** die Kapselwand als Hydrogel, bevorzugt im verfestigten Zustand als Polyelektrolyt-Komplex, realisiert ist.

8. Verfahren nach Anspruch 3, **dadurch gekennzeichnet, dass** das flüssige Kapselwandmaterial aus der Gruppe ausgewählt ist, die aus Alginat, Chitosan, einem anderen Polyelektrolyt oder Polyelektrolytderivat besteht.

9. Verfahren nach einem der Ansprüche 1 bis 8, **dadurch gekennzeichnet, dass** die Kapselwand aus einer Mehrzahl von Lagen, die weiter bevorzugt nacheinander aufgebracht und/oder verfestigt werden, realisiert ist.

10. Verfahren nach einem der Ansprüche 1 bis 9, **dadurch gekennzeichnet, dass** die Mehrzahl von Kapseln sphärisch ist und einen Außendurchmesser aufweist, der im Bereich zwischen 0.1 und 2,5 mm.

11. Verfahren nach einem der Ansprüche 1 bis 10, **dadurch gekennzeichnet, dass** die Mehrzahl von Kapseln eine Kapselwandstärke aufweist, die im Bereich zwischen 30 und 300 Mikrometern, bevorzugt zwischen 50 und 250 Mikrometern, liegt.

12. Vorrichtung zur In-Situ-Extraktion eines mittels eines Mikroorganismus oder einer biologischen Zelle erzeugten Produktes, insbesondere eines Fermentationsproduktes, zur Durchführung des Verfahrens nach einem der Ansprüche 1 bis 11, mit einem Reaktorbehälter (22) zur Aufnahme einer biologischen Zell- bzw. Mikroorganismenkultur in flüssiger Phase sowie einer Substanz, die in Verbindung mit der Zell- bzw. Mikroorganismenkultur das erzeugte Produkt entstehen läßt, **gekennzeichnet durch** außerhalb des Reaktorbehälters (22) vorgesehene Mittel zum Verkapseln eines organischen Lösungsmittels zum Trennen des erzeugten Produktes von der flüssigen Phase in eine organische Phase, wobei die Mittel zum Verkapseln eine Düsenanordnung (14) aus einer ersten, inneren Düse für das organische Lösungsmittel sowie einer zweiten, äußeren und konzentrisch angeordneten Düse für ein flüssiges Kapselwandmaterial aufweisen und diese Doppeldüse mit einem Vibrator mechanisch verbunden ist, um die austretenden Kapseln zu erzeugen, Mittel zum Einbringen von **durch** die Verkapselungsmittel erzeugten Kapseln in den Reaktorbehälter.

13. Vorrichtung nach Anspruch 12, **dadurch gekennzeichnet, dass** die Mittel zum Verkapseln eine außerhalb des Reaktorbehälters vorgesehene, der Düsenanordnung (14) nachgeschaltete und mit einem eine Verfestigung des flüssigen Kapselmaterials bewirkenden Verfestigungsmediums gefüllten Verfestigungseinheit (20) aufweisen.

14. Vorrichtung nach Anspruch 12 oder 13, **gekennzeichnet durch** Mittel zur Re-Extraktion (24) des erzeugten Produktes aus der Mehrzahl von Kapseln.

## Claims

1. Process for the in-situ extraction of a product generated by means of a microorganism or a biological cell, in particular a fermentation product, having the steps:
- providing a biological cell culture or microorganism culture in liquid, preferably aqueous phase in a reaction container;
- adding a substrate, in particular an amino acid, to the liquid phase which is selected and/or formed so that the generated product is produced in the liquid phase by action of the cells or microorganisms, and
- separating the generated product from the liquid phase into an organic phase by means of a solvent,
**characterised in that** the step of separation has the steps:
- encapsulating an organic solvent as a solvent outside of the reaction container into a plurality of capsules having a capsule wall which has a barrier action for the cells or microorganisms and permeability for the generated product, and
- introducing the plurality of capsules into the reaction container and/or into the liquid phase.

2. Process according to claim 1, **characterised by** the steps:
- removing the plurality of capsules from the reaction container after a predetermined time has lapsed after introduction and
- releasing the generated product and/or the organic phase by destroying the capsule wall or using a further solvent.

3. Process according to claim 1 or 2, **characterised in that** the step of encapsulation has the steps:
- discharging the organic solvent through a first nozzle,
- simultaneous discharging of a liquid capsule wall material through a second concentrically arranged nozzle,
- separating this solvent-capsule wall material mixture into equal fractions by superimposing an external vibration so that capsule formation results from organic solvent surrounded by the liquid capsule wall material, and
- solidifying the liquid capsule wall material after capsule formation.

4. Process according to claim 3, **characterised in that** the periodically oscillating change in a frequency range between 30 and 4,000 Hz.

5. Process according to claim 3 or 4, **characterised in that** the step of solidification has the introduction of capsules into a gelling solution effecting a gelling reaction.

6. Process according to one of claims 1 to 5, **characterised by** the autoclaving of the plurality of capsules before introduction into the reaction container.

7. Process according to one of claims 1 to 6, **characterised in that** the capsule wall is realised as a hydrogel, preferably in the solidified state as a polyelectrolyte complex.

8. Process according to claim 3, **characterised in that** the liquid capsule wall material is selected from the group which consists of alginate, chitosan, a different polyelectrolyte or polyelectrolyte derivative.

9. Process according to one of claims 1 to 8, **characterised in that** the capsule wall is realised from a plurality of layers which are also preferably applied and/or solidified one after another.

10. Process according to one of claims 1 to 9, **characterised in that** the plurality of capsules are spherical and have an external diameter which in the range between 0.1 and 2.5 mm.

11. Process according to one of claims 1 to 10, **characterised in that** the plurality of capsules have a capsule wall thickness which lies in the range between 30 and 300 micrometres, preferably between 50 and 250 micrometres.

12. Device for the in-situ extraction of a product generated by means of a microorganism or a biological cell, in particular a fermentation product, for carrying out the process according to one of claims 1 to 11, having a reactor container (22) for receiving a biological cell culture or microorganism culture in liquid phase and a substance allows the generated product to be produced in conjunction with the cell culture or microorganism culture, **characterised by** means for encapsulating an organic solvent provided outside of the reactor container (22), for separating the generated product from the liquid phase into an organic phase, wherein the means for encapsulating have a nozzle arrangement (14) of a first, inner nozzle for the organic solvent and a second, outer and concentrically arrange nozzle for a liquid capsule wall material and this double nozzle is mechanically connected to a vibrator in order to generate the emerging capsules, means for introducing capsules generated by the encapsulating means into the reactor container.

13. Device according to claim 12, **characterised in that** the means for encapsulating have a solidification unit (20) filled with a solidification medium effecting solidification of the liquid encapsulating material and provided outside of the reactor container, downstream of the nozzle arrangement (14).

14. Device according to claim 12 or 13, **characterised by** means for re-extraction (24) of the generated product from the plurality of capsules.

## Revendications

1. Procédé d'extraction in situ d'un produit généré au moyen d'un microorganisme ou d'une cellule biologique, en particulier d'un produit de fermentation, comprenant les étapes suivantes :
- la mise à disposition d'une culture biologique de cellules ou de microorganismes en phase liquide, de préférence aqueuse dans un récipient de réaction ;
- l'adjonction d'un substrat, en particulier d'un acide aminé, à la phase liquide, qui est choisi et/ou formé de telle sorte que, par l'action des cellules ou des microorganismes, le produit généré est produit dans la phase liquide et
- la séparation du produit généré de la phase liquide dans une phase organique au moyen d'un solvant,
**caractérisé en ce que** l'étape de séparation présente les étapes de :
- l'encapsulation d'un solvant organique comme solvant en dehors du récipient de réaction en une pluralité de capsules avec une paroi de capsule qui présente un effet de barrière pour les cellules ou les microorganismes ainsi qu'une perméabilité au produit généré, et
- l'introduction de la pluralité de capsules dans le récipient de réaction et/ou dans la phase liquide.

2. Procédé selon la revendication 1, **caractérisé par** les étapes comprenant :
- le retrait de la pluralité de capsules hors du récipient de réaction après écoulement d'un laps de temps prédéterminé après l'introduction et
- la libération du produit généré et/ou de la phase organique par la destruction de la paroi des capsules ou l'utilisation d'un autre solvant.

3. Procédé selon la revendication 1 ou 2, **caractérisé en ce que** l'étape d'encapsulation présente les étapes comprenant :
- l'évacuation du solvant organique par une première buse,
- l'introduction concomitante d'un matériau liquide de la paroi des capsules par une deuxième buse aménagée de manière concentrique,
- la division de ce mélange de solvant-matériau de la paroi des capsules par superposition d'une oscillation externe, en fractions de même grandeur de sorte que la formation de capsules de solvant organique entouré par le matériau liquide de la paroi de capsules soit réalisée, et
- la solidification du matériau liquide de la paroi des capsules après la formation des capsules.

4. Procédé selon la revendication 3, **caractérisé en ce que** la modification s'effectuant par oscillations périodiques intervient dans une plage de fréquence entre 30 et 4000 Hz.

5. Procédé selon la revendication 3 ou 4, **caractérisé en ce que** l'étape de solidification comprend l'introduction des capsules dans une solution de gélification entraînant une réaction de gélification.

6. Procédé selon l'une quelconque des revendications 1 à 5, **caractérisé par** l'autoclavage de la pluralité de capsules avant l'introduction dans le récipient de réaction.

7. Procédé selon l'une quelconque des revendications 1 à 6, **caractérisé en ce que** la paroi des capsules est réalisée sous la forme d'hydrogel, de préférence à l'état solidifié, sous la forme d'un complexe poly-électrolytique.

8. Procédé selon la revendication 3, **caractérisé en ce que** le matériau liquide de la paroi des capsules est choisi dans le groupe comprenant l'alginate, le chitosan, un autre poly-électrolyte ou dérivé de poly-électrolyte.

9. Procédé selon l'une quelconque des revendications 1 à 8, **caractérisé en ce que** la paroi des capsules est réalisée à partir d'une pluralité de couches qui, de manière davantage préférée, sont réalisées et/ou solidifiées successivement.

10. Procédé selon l'une quelconque des revendications 1 à 9, **caractérisé en ce que** la majorité des capsules est sphérique et présente un diamètre extérieur qui se situe dans une plage entre 0,1 et 2,5 mm.

11. Procédé selon l'une quelconque des revendications 1 à 10, **caractérisé en ce que** la majorité des capsules présente une épaisseur de paroi de capsule qui se situe dans une plage entre 30 et 300 microns, de préférence entre 50 et 250 microns.

12. Dispositif pour l'extraction in situ d'un produit généré au moyen d'un microorganisme ou d'une cellule biologique, en particulier d'un produit de fermentation, pour réaliser le procédé selon l'une quelconque des revendications 1 à 11, avec un récipient de réaction (22) destiné à recevoir une culture biologique de cellules ou de microorganismes en phase liquide ainsi qu'une substance qui permet de produire le produit généré en relation avec la culture de cellules ou de microorganismes, **caractérisé par** les moyens prévus, en dehors du récipient de réaction, pour l'encapsulation d'un solvant organique pour séparer le produit généré de la phase liquide, en une phase organique, les moyens d'encapsulation comprenant un assemblage de buses (14) à partir d'une première buse intérieure pour le solvant organique ainsi que d'une deuxième buse extérieure et aménagée de manière concentrique pour un matériau de la paroi des capsules, et cette buse double étant reliée de manière mécanique à un dispositif de vibrations pour produire les capsules en sortant, des moyens d'introduction des capsules produites par les moyens d'encapsulation, dans le récipient de réaction.

13. Dispositif selon la revendication 12, **caractérisé en ce que** les moyens d'encapsulation comprennent une unité de solidification (20) prévue en dehors du récipient de réaction, reliée en aval de l'assemblage de buses (14) et remplie d'un milieu de solidification entraînant une solidification du matériau liquide des capsules.

14. Dispositif selon la revendication 12 ou 13, **caractérisé par** des moyens de re-extraction (24) du produit généré à partir de la pluralité de capsules.
